# EUROPÄISCHE PATENTANMELDUNG

(11) **EP 3 461 419 A1**
(43) Veröffentlichungstag der Anmeldung: **03.04.2019**
(21) Anmeldenummer: 17194033.1
(22) Anmeldetag: 29.09.2017
(51) Int. Cl.: A61B 8/00, A61B 34/30, A61B 90/00

(54) **ANSTEUERUNGSVERFAHREN FÜR EIN ROBOTISCHES GERÄT MIT EINER ULTRASCHALLVORRICHTUNG UND ROBOTISCHES GERÄT**

(71) Anmelder: Siemens Healthcare GmbH, 91052 Erlangen (DE)
(72) Erfinder: Fischer, Peter, 91058 Erlangen (DE); Mewes, Philip, 90439 Nürnberg (DE); Mönnich, Holger, 86316 Friedberg (DE); Müller, Gunter, 90562 Heroldsberg (DE)

(57) **Zusammenfassung**

Verfahren zur Ansteuerung eines robotischen Geräts, dessen an einer kinematischen Kette angeordneter Endeffektor mit einem Objekt interagiert, und mit einer zu dem robotischen Gerät registrierten Ultraschallvorrichtung zur Bewegungserfassung des Objekts mit den folgenden Schritten: Erfassung von Ultraschallbilddaten des Objekts, Bestimmung der relativen Bewegung des Objekts anhand der Ultraschallbilddaten unter Verwendung eines mathematischen Verfahrens, Bestimmung der absoluten Bewegung durch Registrierung zu einem Planungsdatensatz, und Kompensation von Bewegungen des Objekts im Rahmen der Interaktion des Endeffektors des robotischen Geräts mit dem Objekt, wobei auf der Basis der aktuellen Bewegungsbestimmung zukünftige Bewegungen des Objekts und eine daran angepasste Positionierung des Endeffektors des robotischen Geräts vorausberechnet werden und das robotische Gerät entsprechend angesteuert wird, wobei bei der Berechnung der angepassten Positionierung des Endeffektors des robotischen Geräts eine bezüglich der Bewegung des Objekts zeitliche Verzögerung eingerechnet wird

## Beschreibung

Die Erfindung betrifft ein Ansteuerungsverfahren für ein robotisches Gerät mit einer Ultraschallvorrichtung gemäß dem Patentanspruch 1 sowie ein robotisches Gerät zur Durchführung eines derartigen Verfahrens gemäß dem Patentanspruch 8.

Im Bereich der robotisch-chirurgischen Eingriffe, welche sowohl diagnostischer als auch therapeutischer Natur sein können, und bei welchen ein Objekt von einem robotischen Gerät angesteuert wird oder eine Interaktion mit dem Objekt durchgeführt wird, gilt es, stets eine größtmögliche Präzision zu erreichen. Ein Beispiel hierfür ist z.B. in der roboterunterstützten Wirbelsäulenchirurgie zu finden, bei welcher von einem robotischen Gerät eine Bohrhülse gehalten wird, durch welche eine Schraube oder ein anderes Instrument in einen Knochen des Patienten eingeführt wird.

Bekannte robotische Systeme und Verfahren in diesem Bereich arbeiten deshalb häufig mit externen optischen Tracking Systemen, welche z.B. eine Kamera und Marker aufweisen, um Bewegungen des Objekts (z.B. Atembewegung oder Herzbewegung bei einem Patienten) genau aufzeichnen zu können. Die Marker werden physikalisch an dem Objekt befestigt und dann direkt mit der Kamera verfolgt, wobei eine direkte Sichtverbindung notwendig ist. Auf diese Weise können die Bewegungen des Objekts optisch nachverfolgt werden. Das Anbringen der Marker an dem Objekt und das Erfordernis einer unverstellten Sichtverbindung für die Kamera sind jedoch häufig einschränkend und unerwünscht. Auch andere Systeme zur Nachverfolgung von Patientenbewegungen wie EMT Tracking oder Live-Fluoroskopie haben Nachteile, behindern den Workflow, sind invasiv oder bringen Risiken durch unnötige Röntgenstrahlung.

Aus der nicht vorveröffentlichten europäischen Patentanmeldung mit dem Anmeldeaktenzeichen 17159352.8 ist ein System und ein Verfahren bekannt, bei welchem zur Bewegungserfassung eine 3D-Ultraschallvorrichtung z.B. direkt an einem robotischen Gerät angeordnet ist, welche die Bewegungen des Objekts in allen drei Dimensionen erfasst. Zusätzlich zu der Bewegungserfassung ist jedoch noch eine Ansteuerung des robotischen Geräts notwendig, um ermittelte Bewegungen mit dem robotischen Gerät exakt und für den Patienten gefahrlos ausgleichen zu können.

Es ist Aufgabe der vorliegenden Erfindung, ein Verfahren bereitzustellen, welches eine Ansteuerung eines robotischen Geräts mit einer Ultraschallvorrichtung durchführt, durch welche eine präzise Erfassung und Kompensation von Bewegungen eines Objekts möglich ist; des Weiteren ist es Aufgabe der Erfindung, ein für die Durchführung des Verfahrens geeignetes robotisches Gerät bereitzustellen.

Die Aufgabe wird erfindungsgemäß gelöst durch ein Ansteuerungsverfahren für ein robotisches Gerät mit einer Ultraschallvorrichtung gemäß dem Patentanspruch 1 und von einem robotischen Gerät gemäß dem Patentanspruch 8. Vorteilhafte Ausgestaltungen der Erfindung sind jeweils Gegenstand der zugehörigen Unteransprüche.

Erfindungsgemäß ist ein Verfahren zur Ansteuerung eines robotischen Geräts, dessen an einer kinematischen Kette angeordneter Endeffektor mit einem Objekt interagiert, und mit einer zu dem robotischen Gerät registrierten Ultraschallvorrichtung zur Bewegungserfassung des Objekts mit den folgenden Schritten vorgesehen: Erfassung von Ultraschallbilddaten des Objekts, Bestimmung der relativen Bewegung des Objekts anhand der Ultraschallbilddaten unter Verwendung eines mathematischen Verfahrens, Bestimmung der absoluten Bewegung durch Registrierung zu einem Planungsdatensatz, und Kompensation von Bewegungen des Objekts im Rahmen der Interaktion des Endeffektors des robotischen Geräts mit dem Objekt, wobei auf der Basis der aktuellen Bewegungsbestimmung zukünftige Bewegungen des Objekts und eine daran angepasste Positionierung des Endeffektors des robotischen Geräts vorausberechnet werden und das robotische Gerät entsprechend angesteuert wird, wobei bei der Berechnung der angepassten Positionierung des Endeffektors des robotischen Geräts eine bezüglich der Bewegung des Objekts zeitliche Verzögerung eingerechnet wird. Das erfindungsgemäße Verfahren ermöglicht eine präzise Kompensierung von Live-Bewegungen des Objekts derart, dass Gefahren z.B. durch Verletzungen für das Objekt bzw. den Patienten minimiert werden können. Hintergrund der Erfindung ist die Erkenntnis, dass verschiedene Vorgänge, z.B. die Bewegungserfassung selbst, aber auch das Ansprechen des robotischen Geräts auf ein Steuersignal eine gewisse Zeit zur Ausführung benötigen. Um die Kompensierung so präzise wie möglich durchzuführen, werden zukünftige Bewegungen des Objekts und geplante Bewegungen des robotischen Geräts unter Berücksichtigung der zeitlichen Verzögerung berechnet. Anstatt der Bewegung des robotischen Gerätes mit zeitlicher Verzögerung "hinterherzufahren", wie aus dem Stand der Technik bekannt, wird die zukünftige Bewegung des Objekts vorausberechnet und vom robotischen Gerät "abgepasst".

Meist handelt es sich bei den Bewegungen des Objekts um eine periodische Bewegung, z.B. global um Atmung oder Herzschlag des Objekts (Patienten) oder lokal um eine Bewegung, die beschleunigt oder abbremst.

Nach einer Ausführung der Erfindung werden bei der Bestimmung bzw. Abschätzung der zeitlichen Verzögerung zumindest die Dauer der Erfassung der Ultraschallbilddaten, die Dauer der Bestimmung der absoluten Bewegung und die Reaktionszeit des robotischen Geräts berücksichtigt. Dies sind drei Hauptfaktoren, welche zur zeitlichen Verzögerung beitragen. Es können auch weitere Faktoren berücksichtigt werden, um die tatsächliche Verzögerung zu bestimmen, z.B. die Dauer der Vorabberechnungen der Positionen von Objekt und robotischem Gerät.

Nach einer weiteren Ausführung der Erfindung werden im Rahmen des mathematischen Verfahrens zur Bestimmung der relativen Bewegung des Objekts Merkmale aus Bilddaten extrahiert; diese können dann z.B. räumlich auf Merkmale weiterer Bilder transformiert werden. So kann z.B. das mathematische Verfahren zur Bestimmung der relativen Bewegung des Objekts die Bildbearbeitungsverfahren Feature Extraction oder optischen Fluss verwenden. Hierbei handelt es sich um zwei mögliche bekannte Verfahren, um Informationen aus Bilddaten auszulesen. Es können auch andere Bildbearbeitungsverfahren verwendet werden.

Zweckmäßigerweise für die Ausführung eines sich über einen längeren Zeitraum erstreckenden Eingriffs wird bis zu einem Abbruchkriterium eine fortlaufende Bewegungserfassung und Bewegungskompensation durchgeführt.

In vorteilhafter Weise für eine besonders einfache und schnelle Bewegungsberechnung wird die Ultraschallvorrichtung zu einem Patientenkoordinatensystem registriert.

Nach einer weiteren Ausführung der Erfindung werden die vorausberechneten Positionen des Endeffektors des robotischen Geräts gefiltert und/oder aufsynchronisiert. Diese beiden Verfahren dienen vor allem dazu, Messrauschen und Messfehler zu vermeiden sowie plötzliche und vom normalen Bewegungsfluß abweichende Bewegungen des Objekts, wie z.B. Zuckungen, mit dem robotischen Gerät zu vermeiden bzw. solchen Bewegungen nach Möglichkeit nicht zu folgen. Als Beispiel für einen geeigneten, bekannten Filter wird der Kalman-Filter angewandt.

Um die zeitliche Verzögerung zu kompensieren muss im Allgemeinen das robotische Gerät zunächst deutlich stärker beschleunigen, um mit der Bewegung des Objekts synchron zu werden als dies das Objekt selbst tut, gleichzeitig muss das robotische Gerät rechtzeitig abbremsen um nicht über das Ziel hinauszuschießen.

Zur Ausführung des erfindungsgemäßen Verfahrens wird ein robotisches Gerät mit einer zu dem robotischen Gerät registrierten Ultraschallvorrichtung, das robotische Gerät aufweisend eine kinematischen Kette von bewegbaren Komponenten, einen am Ende der kinematischen Kette angeordneten Endeffektor, welche zur Interaktion mit einem Objekt ausgebildet ist, eine Steuerungseinrichtung zur Steuerung der kinematischen Kette und der Ultraschalleinrichtung, eine Bildbearbeitungseinrichtung und eine Berechnungseinheit, wobei die Ultraschalleinrichtung dazu ausgebildet ist, Ultraschallbilddaten des Objekts zu erfassen, die Bildbearbeitungseinrichtung und die Berechnungseinheit zusammenwirkend dazu ausgebildet sind, aus den Ultraschallbilddaten und weiteren Daten die relative Bewegung und die absolute Bewegung des Objekts zu bestimmen, auf der Basis der aktuellen Bewegungsbestimmung zukünftige Bewegungen des Objekts zu berechnen sowie eine daran angepasste Positionierung des robotischen Geräts mit einer bezüglich der Bewegung des Objekts zeitlichen Verzögerung eingerechnet vorauszuberechnen und die Steuerungseinrichtung dazu ausgebildet ist, die Positionierung des robotischen Geräts anzusteuern, verwendet.

Nach einer Ausführung der Erfindung weist das robotische Gerät einen Endeffektor in Form einer Bohrhülse auf. Dies ist insbesondere im Zusammenhang mit Eingriffen zum Einbringen bzw. Einbohren von Schrauben oder anderen Implantaten in Knochenstrukturen wichtig.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: ein robotisches Gerät mit Ultraschallvorrichtung gemäß dem Stand der Technik;
- FIG 2: eine bekannte zeitliche Abfolge der Bewegung von Objekt und robotischem Gerät;
- FIG 3: eine zeitliche Abfolge unter Verwendung der erfindungsgemäßen Bewegungskompensation mit Synchronisierung durch Vorhersage der Bewegung;
- FIG 4: eine Abfolge des erfindungsgemäßen Verfahrens; und
- FIG 5: ein erfindungsgemäßes robotisches Gerät.

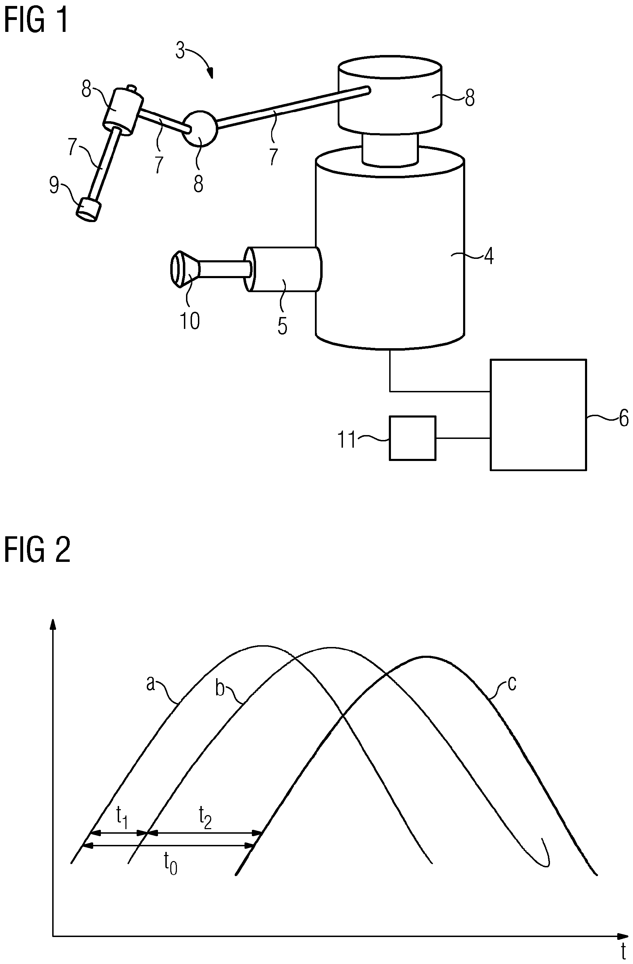

In der FIG 1 ist ein bekanntes robotisches Gerät 4 mit einer Ultraschallvorrichtung 5 zur Bewegungserfassung gezeigt, wie es zum Beispiel in der nicht vorveröffentlichten europäischen Patentanmeldung mit dem Anmeldeaktenzeichen 17159352.8 beschrieben ist. Das robotische Gerät 4 weist einen Roboterarm 3 auf, welcher mehrere (mindestens 3) Segmente 7 und Gelenke 8 und am freien Ende einen Endeffektor 9 aufweist. Der Endeffektor 9 ist zur Interaktion mit einem Objekt, insbesondere einem Patienten, ausgebildet. Ein Beispiel für den Einsatz eines derartigen robotischen Gerätes ist in der roboterunterstützten Wirbelsäulenchirurgie zu finden. Hier ist der Endeffektor z.B. als Bohrhülse ausgebildet, welche von dem Roboterarm gehalten wird. Die Bohrhülse wird auf den Knochen eines Patienten aufgelegt und durch die Bohrhülse wird von einem Chirurgen eine Schraube oder ein anderes Instrument in den Knochen des Patienten eingeführt. Die Ultraschallvorrichtung 5 ist z.B. an dem robotischen Gerät 4 angeordnet oder anders mit diesem mechanisch verbunden und besitzt einen Ultraschallkopf 10, welcher zur Aufzeichnung von (3D)-Ultraschallbilddaten ausgebildet ist. Der Ultraschallkopf 10 wird dazu in Kontakt mit dem Objekt gebracht. Zur Ansteuerung des robotischen Geräts 4 und der Ultraschallvorrichtung ist eine Steuerungseinrichtung 6 vorgesehen, zudem kann eine Bildbearbeitungseinrichtung 11 zur Bearbeitung der Ultraschallbilddaten vorhanden sein. Im Fall einer Bohrhülse kann der Ultraschallkopf auch z.B. in dieser integriert sein.

In der FIG 2 ist mittels einer Zeitachse t gezeigt, wie üblicherweise eine Bewegungsverzögerung zu Stande kommt. So erfolgt z.B. eine zyklische Bewegung a des Objekts (z.B. Atembewegung oder Herzbewegung eines Patienten). Nach einem ersten Zeitraum t₁ sind die Ultraschallbilddaten erfasst und ausgewertet b und nach einem zweiten Zeitraum t₂ (z.B. Reaktionszeit robotisches Gerät) erfolgt erst die Bewegung c des robotischen Geräts. Insgesamt ergibt sich eine zeitliche Verzögerung t₀ zwischen der Bewegung a des Objekts und der Bewegung des robotischen Geräts c, so dass eine eventuelle darauf basierende Bewegungskompensation keine hohe Qualität aufweist. Gerade im chirurgischen Umfeld entsteht durch die schlechte Qualität eine Gefährdung von Patienten z.B. durch Verletzungen, so dass stattdessen eine qualitativ hochwertige Bewegungskompensation notwendig ist.

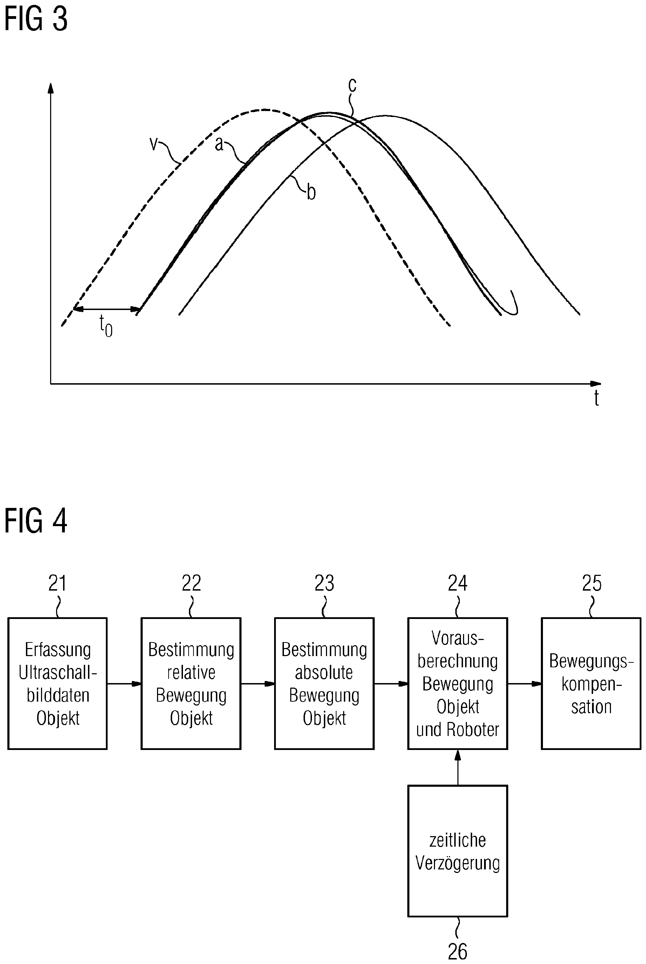

In der FIG 4 ist ein Ablauf eines erfindungsgemäßen Verfahrens zur Ansteuerung eines mit einem Objekt interagierenden robotischen Geräts mit einer zu dem robotischen Gerät registrierten Ultraschallvorrichtung zur Bewegungserfassung des Objekts gezeigt. Das erfindungsgemäße Verfahren bewirkt eine präzise und hochqualitative Bewegungskompensation und kann somit auch im chirurgischen Umfeld eingesetzt werden. Auf der Basis der Erkenntnis, dass verschiedene Vorgänge, z.B. die Bewegungserfassung selbst, aber auch das Ansprechen des robotischen Geräts auf ein Steuersignal eine gewisse Zeit zur Ausführung benötigen, wird die entsprechende benötigte Zeit als zeitliche Verzögerung in die geplante Bewegung des robotischen Geräts eingerechnet und mit zukünftigen vorausberechneten Bewegungen des Objekts abgeglichen.

Die Ultraschallvorrichtung ist bevorzugt zu einem Objektkoordinatensystem (z.B. Patientenkoordinatensystem) registriert. Unter einer Bewegung des robotischen Geräts ist hierbei im Allgemeinen eine Bewegung des Roboterarms zur Positionierung des Endeffektors zu verstehen.

In einem ersten Schritt 21 werden mittels der Ultraschallvorrichtung 5 Ultraschallbilddaten eines sich bewegenden Objekts (z.B. ein Patient) aufgenommen. Der Ultraschallkopf muss dabei im Kontakt mit dem Objekt stehen. Bevorzugt handelt es sich dabei um mindestens zwei oder eine Vielzahl von Ultraschallbildern. Diese können 2D- oder 3D-Ultraschallbilddaten sein. Bei der Bewegung des Objektes kann es sich z.B. um die zyklische Atembewegung oder Herzbewegung eines Patienten handeln.

In einem zweiten Schritt 22 wird die relative Bewegung des Objekts anhand der aufgenommenen Ultraschallbilddaten unter Verwendung eines mathematischen Verfahrens bestimmt bzw. berechnet. So werden z.B. zur Bestimmung der relativen Bewegung des Objekts aus den Ultraschallbilddaten Merkmale des Objekts extrahiert. Beispiele für dafür nutzbare Verfahren kann z.B. die sogenannte Feature Extraction oder der sogenannte Optische Fluss sein. Es gibt viele weitere Möglichkeiten, eine derartige Bewegungsdetektion aus den Ultraschallbilddaten zu entnehmen.

In einem dritten Schritt 23 wird die absolute Bewegung durch Registrierung zu einem Planungsdatensatz bestimmt. Unter der absoluten Bewegung wird hier insbesondere die zeitlich eingeordnete Bewegung verstanden. Unter einem Planungsdatensatz wird insbesondere ein zuvor bestehender initialer Datensatz, z.B. 2D- oder 3D-Datensatz verstanden. Es kann sich dabei um einen Ultraschalldatensatz handeln, wahlweise können jedoch auch andere Datensätze verwendet werden, z.B. Röntgenbilddaten. Es können z.B. aus dem zweiten Schritt 22 erzeugte Vektoren oder Bildpunkte erhalten, passend gefiltert und im dritten Schritt zu einem vorhergehenden Bilddatensatz oder zu einem bestehenden initialen 3D oder 2D Datensatz registriert werden. Das Ergebnis der Berechnungen kann z.B. eine neue Pose im Raum oder ein Geschwindigkeitsvektor sein, welche die Bewegung des Objektes (Patienten) an einem definierten Punkt im Volumen des Objektes (Patienten) widerspiegelt.

In einem vierten Schritt 24 wird anschließend eine zeitliche Verzögerung in die geplante Bewegung des robotischen Geräts eingerechnet und mit vorausberechneten zukünftigen Bewegungen des Objekts synchronisiert. Die notwendige zeitliche Verzögerung kann z.B. zuvor in einem Zusatzschritt 26 abgeschätzt, berechnet oder empirisch ermittelt worden sein, im Allgemeinen wird hier ein konstanter Wert verwendet. Bevorzugt sind in der zeitlichen Verzögerung die Aufnahme der Ultraschallbilddaten, die Ermittlungen der Bewegung des Objekts und die Ansteuerzeit des robotischen Geräts in ihrer Dauer berücksichtigt. Zusätzlich kann auch die Dauer für die Vorausberechnung der Objektbewegung und der geplanten Bewegung des robotischen Geräts enthalten sein. Zusätzlich können dynamische Limitierungen der Bewegung des robotischen Geräts bei der Planung der Bewegungskompensation berücksichtigt werden. So kann das robotische Gerät z.B. in manchen Fällen (z.B. Zuckungen) nicht so schnell beschleunigen wie der Patient oder eine Bewegung muss aufsynchronisiert werden, z.B. bei Atmung oder Herzschlag, um Ruckeln zu vermeiden. Um Bewegungen denen nicht gefolgt werden kann (Zuckung) oder Messrauschen nicht mit vorherzusagen, werden die geplanten Bewegungen des robotischen Geräts im Allgemeinen gefiltert (z.B. mit dem sogenannten Kalman Filter), um Messfehler zu reduzieren.

Insgesamt wird also um die zeitliche Verzögerung auszugleichen, eine Position für das robotische Gerät vorhergesagt. Zugrunde liegt hierbei die Bestimmung der Bewegung des Objekts aus der Bewegungserfassung. Meist handelt es sich um eine periodische Bewegung, z.B. global um Atmung oder Herzschlag des Objekts (Patienten) oder lokal um eine Bewegung, die beschleunigt oder abbremst.

In einem fünften Schritt 25 wird die Bewegung des Objektes durch das robotische Gerät kompensiert. Es kann dabei notwendig sein, dass das robotische Gerät deutlich mehr als bei der zu kompensierenden Bewegung des Objekts beschleunigen muss, um synchron zu werden und auch mehr abbremsen muss, um nicht über das geplante Ziel hinauszufahren.

In FIG 3 ist gezeigt, wie bei Verwendung des erfindungsgemäßen Verfahrens die Bewegungen entlang der Zeitachse t aussehen. Die Bewegung c des robotischen Geräts ist nun im Rahmen einer Fehlertoleranz synchron zu der Bewegung des Objekts a, die Vorausberechnung V für das robotische Gerät berücksichtigt die zeitliche Verzögerung t₀.

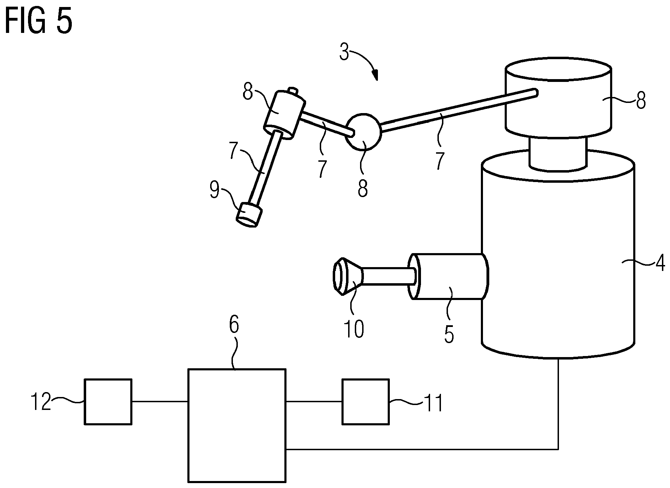

In der FIG 5 ist ein erfindungsgemäßes robotisches Gerät 4 mit einer zu dem robotischen Gerät registrierten Ultraschallvorrichtung 5 gezeigt. Das robotische Gerät 4 weist einen Roboterarm 3 auf, welcher mehrere (mindestens drei) Segmente 7 und Gelenke 8 und am freien Ende einen Endeffektor 9, zum Beispiel eine Bohrhülse, aufweist. Der Endeffektor 9 ist elche zur Interaktion mit einem Objekt, z.B. einem Patienten oder einem Organ des Patienten, ausgebildet. Das robotische Gerät weist eine Steuerungseinrichtung 6 zur Steuerung des Roboterarms und der Ultraschallvorrichtung 5 auf. Außerdem weist das robotische Gerät eine Bildbearbeitungseinrichtung 11 und eine Berechnungseinheit 12 auf. Die Ultraschalleinrichtung ist ausgebildet, Ultraschallbilddaten des Objekts zu erfasse. Die Bildbearbeitungseinrichtung 11 und die Berechnungseinheit 12 sind zusammenwirkend dazu ausgebildet, aus den Ultraschallbilddaten und weiteren Daten (zuvor aufgenommenen Bilddaten oder z.B. Planungsdaten) die relative Bewegung und die absolute Bewegung des Objekts zu bestimmen, auf der Basis der aktuellen Bewegungsbestimmung zukünftige Bewegungen des Objekts zu berechnen sowie eine daran angepasste Positionierung des robotischen Geräts mit einer bezüglich der Bewegung des Objekts zeitlichen Verzögerung eingerechnet vorauszuberechnen. Die Steuerungseinrichtung 6 ist dazu ausgebildet, die Positionierung des Endeffektors des robotischen Geräts anzusteuern.

Die Erfindung lässt sich in folgender Weise kurz zusammenfassen: Für einen für ein Objekt (z.B. Patienten) besonders sicheren medizinischen Eingriff mit einem robotischen Gerät ist ein Verfahren zur Ansteuerung eines robotischen Geräts, dessen an einer kinematischen Kette angeordneter Endeffektor mit einem Objekt interagiert, und mit einer zu dem robotischen Gerät registrierten Ultraschallvorrichtung zur Bewegungserfassung des Objekts mit den folgenden Schritten vorgesehen: Erfassung von Ultraschallbilddaten des Objekts, Bestimmung der relativen Bewegung des Objekts anhand der Ultraschallbilddaten unter Verwendung eines mathematischen Verfahrens, Bestimmung der absoluten Bewegung durch Registrierung zu einem Planungsdatensatz, und Kompensation von Bewegungen des Objekts im Rahmen der Interaktion des Endeffektors des robotischen Geräts mit dem Objekt, wobei auf der Basis der aktuellen Bewegungsbestimmung zukünftige Bewegungen des Objekts und eine daran angepasste Positionierung des Endeffektors des robotischen Geräts vorausberechnet werden und das robotische Gerät entsprechend angesteuert wird, wobei bei der Berechnung der angepassten Positionierung des Endeffektors des robotischen Geräts eine bezüglich der Bewegung des Objekts zeitliche Verzögerung eingerechnet wird.

## Patentansprüche

1. Verfahren zur Ansteuerung eines robotischen Geräts, dessen an einer kinematischen Kette angeordneter Endeffektor mit einem Objekt interagiert, und mit einer zu dem robotischen Gerät registrierten Ultraschallvorrichtung zur Bewegungserfassung des Objekts mit den folgenden Schritten:
• Erfassung von Ultraschallbilddaten des Objekts,
• Bestimmung der relativen Bewegung des Objekts anhand der Ultraschallbilddaten unter Verwendung eines mathematischen Verfahrens,
• Bestimmung der absoluten Bewegung durch Registrierung zu einem Planungsdatensatz, und
• Kompensation von Bewegungen des Objekts im Rahmen der Interaktion des Endeffektors des robotischen Geräts mit dem Objekt, wobei auf der Basis der aktuellen Bewegungsbestimmung zukünftige Bewegungen des Objekts und eine daran angepasste Positionierung des Endeffektors des robotischen Geräts vorausberechnet werden und das robotische Gerät entsprechend angesteuert wird, wobei bei der Berechnung der angepassten Positionierung des Endeffektors des robotischen Geräts eine bezüglich der Bewegung des Objekts zeitliche Verzögerung eingerechnet wird.

2. Verfahren nach Anspruch 1, wobei die zeitliche Verzögerung zumindest die Dauer der Erfassung der Ultraschallbilddaten, der Bestimmung der absoluten Bewegung und der Reaktionszeit des robotischen Geräts berücksichtigt.

3. Verfahren nach Anspruch 1 oder 2, wobei im Rahmen des mathematischen Verfahrens zur Bestimmung der relativen Bewegung des Objekts aus den Ultraschallbilddaten Merkmale des Objekts extrahiert werden.

4. Verfahren nach einem der vorangehenden Ansprüche, wobei bis zu einem Abbruchkriterium fortlaufende Bewegungserfassung und Bewegungskompensation durchgeführt wird.

5. Verfahren nach einem der vorangehenden Ansprüche, wobei die Ultraschallvorrichtung zu einem Patientenkoordinatensystem registriert wird.

6. Verfahren nach einem der vorangehenden Ansprüche, wobei die vorausberechneten Positionen der kinematischen Kette des robotischen Geräts gefiltert werden.

7. Verfahren nach einem der vorangehenden Ansprüche, wobei die vorausberechneten Positionen des Endeffektors des robotischen Geräts aufsynchronisiert werden.

8. Robotisches Gerät (4) mit einer zu dem robotischen Gerät registrierten Ultraschallvorrichtung (5), das robotische Gerät (4) aufweisend eine kinematischen Kette von bewegbaren Komponenten, einen am Ende der kinematischen Kette angeordneten Endeffektor (9), welche zur Interaktion mit einem Objekt ausgebildet ist, eine Steuerungseinrichtung (6) zur Steuerung der kinematischen Kette und der Ultraschallvorrichtung (5), eine Bildbearbeitungseinrichtung (11) und eine Berechnungseinheit (12), wobei die Ultraschalleinrichtung (5) dazu ausgebildet ist, Ultraschallbilddaten des Objekts zu erfassen, die Bildbearbeitungseinrichtung (11) und die Berechnungseinheit (12) zusammenwirkend dazu ausgebildet sind, aus den Ultraschallbilddaten und weiteren Daten die relative Bewegung und die absolute Bewegung des Objekts zu bestimmen, auf der Basis der aktuellen Bewegungsbestimmung zukünftige Bewegungen des Objekts zu berechnen sowie eine daran angepasste Positionierung der kinematischen Kette des robotischen Geräts (4) mit einer bezüglich der Bewegung des Objekts zeitlichen Verzögerung eingerechnet vorauszuberechnen und die Steuerungseinrichtung (6) dazu ausgebildet ist, die Positionierung der kinematischen Kette des robotischen Geräts (4) anzusteuern.

9. Robotisches Gerät nach Anspruch 8, welches einen Endeffektor (9) in Form einer Bohrhülse aufweist.
